# EUROPEAN PATENT SPECIFICATION

(11) **EP 3 965 863 B1**
(45) Date of publication and mention of the grant of the patent: **14.01.2026**
(21) Application number: 20743034.9
(22) Date of filing: 30.06.2020
(51) Int. Cl.: A61M 25/09, A61M 25/00

(54) **TORQUE ACCESSORY FOR SUPPORT CATHETER**
DREHMOMENTZUBEHÖR FÜR STÜTZKATHETER
ACCESSOIRE DE COUPLE POUR CATHÉTER DE SUPPORT

(30) Priority: 01.07.2019 US 201962869531 P
(43) Date of publication of application: 16.03.2022
(73) Proprietor: Boston Scientific Limited, Hamilton HM11 (BM)
(72) Inventor: BADADAMATH, Sharath, 583211 Hosapete, Karnataka (IN); DHANOTIYA, Aditya, 452001 Indore (IN)
(74) Representative: Vossius & Partner Patentanwälte Rechtsanwälte mbB
(86) International application number: PCT/US2020/040322
(87) International publication number: WO 2021/003169

(56) References cited:
- US-A- 5 755 695
- US-A1- 2009 259 200
- US-A1- 2010 022 917
- US-A1- 2014 276 225

## Description

### Technical Field

The disclosure pertains to medical devices and more particularly to devices for arterial or vascular intervention techniques, and methods for using such medical devices.

### Background

A wide variety of medical devices have been developed for medical use including, for example, medical devices utilized to treat chronic total occlusion (CTO) and peripheral arterial disease (PAD). In these procedures a support catheter may be used to provide support and column strength to the guidewire. During the procedure, the support catheter hub is often torqued while holding it in the user's primary hand, while the user simultaneously clutches the guidewire with the little finger to avoid torqueing the guidewire. The user torques the catheter shaft using the opposite hand with a pinch grip. Clutching the guidewire with the little finger is difficult due to the small diameter and hydrophilic nature of the guidewire, and with procedure times of 15-20 minutes, this often results in muscle strain. Holding the guidewire in this manner may result in kinking of the guidewire and/or catheter shaft. There is an ongoing need to provide alternative medical devices for these procedures as well as alternative methods for manufacturing and using the medical devices.

US 2009/259200 A1 shows a hemostasis valve apparatus having a quick release apparatus that allows for the exchange of guidewires and other medical instruments into a patient's vasculature while controlling the flow of blood during the exchange. The quick release capability allows tools and instruments to be introduced and removed from the patient's body in a quick and efficient manner without requiring undue attention or manipulation. The quick release capability comprises a lever mechanism which selectively seals or unseals a lumen of the hemostasis valve apparatus. The hemostasis valve apparatus can include a supplemental securement valve assembly which provides a secondary mechanism to secure an instrument positioned in the hemostasis valve and/or control the flow of blood from during the procedure.

US 5 755 695 A shows a steering handle for guiding a medical guidewire and a catheter through a patient's vessels to a treatment site and a method of using the steering handle. The steering handle may be selectively converted to guide the guidewire and catheter either independently or together as a fixed unit.

US 2014/276225 A1 shows a torque device for attaching to and selectively gripping or securing and releasing a guidewire to permit rotational and longitudinal manipulation of the guidewire to steer and control the guidewire.

US 2010/022917 A1 shows a guide wire placement and removal tool that includes a handle mounted to a shaft connected to a wire engagement mechanism disposed opposite the handle. The mechanism includes a housing aligned with the shaft to enable the wire to pass therethrough, and a moveable engagement member biased into a locking position within the housing to engage a wire positioned therein. The mechanism also includes a release member that can be selectively engaged with the engagement member to disengage the engagement member from the wire to allow the wire to move with regard to the tool. Once the release member is released, the engagement member is biased back into a locking engagement with the wire to securely hold the wire within the tool.

### Summary

This disclosure provides design, material, manufacturing method, and use alternatives for medical devices.

In a first example, a guidewire control device comprises a housing having a passage for receiving a guidewire, a clutch including first and second opposing grips biased in a release configuration, the clutch being connected to the housing and actuatable to selectively grip and release the guidewire disposed in the passage, and an interface rotatably connected to the housing, the interface configured to receive a catheter hub and torque the catheter hub.

In addition or alternatively, and in another example, the clutch incudes an engagement member configured to grip the guidewire and prevent rotation of the guidewire while the interface rotates.

In addition or alternatively, and in another example, the engagement member includes first and second opposing pads configured to be moved toward each other and into contact with the guidewire.

In addition or alternatively, and in another example, the clutch further includes first and second opposing grips attached to the first and second opposing pads, respectively, the first and second opposing grips configured to move between a release configuration and a gripping configuration, wherein when in the release configuration, the first and second opposing grips extend radially outward from an outer surface of the housing, and when in the gripping configuration, the first and second opposing grips are disposed radially inward toward the passage.

In addition or alternatively, and in another example, the interface includes a proximal end defining a lumen for receiving the guidewire, the proximal end having at least one protrusion configured to rotate within at least one channel on an inner surface of the passage of the housing.

In addition or alternatively, and in another example, the interface includes a distal end defining a cavity having internal threading configured to receive an externally threaded proximal end of the catheter hub.

In addition or alternatively, and in another example, the guidewire control device further comprises an accessory device including an actuator having a lumen configured to receive a catheter, the actuator having a distal end defining an actuatable clamp configured to engage the catheter, the accessory device further including a sleeve configured to actuate the actuatable clamp.

In addition or alternatively, and in another example, the actuatable clamp includes a plurality of deflectable arms moveable between a release configuration in which the catheter slides through the lumen, and a gripping configuration in which the plurality of deflectable arms engage the catheter and prevent the catheter from moving axially or rotationally.

In addition or alternatively, and in another example, the sleeve defines a proximal chamber configured to receive the plurality of deflectable arms in the release configuration, a central chamber configured to receive the plurality of deflectable arms in the gripping configuration, and a lumen configured to receive the catheter, wherein when the plurality of deflectable arms of the actuator are moved from the proximal chamber into the central chamber, the plurality of deflectable arms are compressed radially inward into engagement with the catheter.

In another example, a guidewire control assembly according to claim is disclosed.

In addition or alternatively, and in another example, the clutch incudes an engagement member configured to grip the guidewire and prevent rotation of the guidewire while the distal housing rotates, the engagement member including first and second opposing pads configured to be moved toward each other and into contact with the guidewire.

In addition or alternatively, and in another example, the clutch further includes first and second opposing grips attached to the first and second opposing pads, respectively, the first and second opposing grips configured to move between a release configuration and a gripping configuration, wherein when in the release configuration, the first and second opposing grips extend radially outward from an outer surface of the proximal housing, and when in the gripping configuration, the first and second opposing grips are disposed radially inward toward the passage.

In addition or alternatively, and in another example, the first and second opposing grips are biased in the release configuration.

In addition or alternatively, and in another example, the distal housing includes a proximal end defining a lumen for receiving the guidewire, the proximal end having at least one protrusion configured to rotate within at least one channel on an inner surface of the passage of the distal housing.

In addition or alternatively, and in another example, the distal housing includes a distal end defining a cavity having internal threading configured to receive an externally threaded proximal end of the catheter hub.

In addition or alternatively, and in another example, the actuatable clamp includes a plurality of deflectable arms moveable between a release configuration in which the catheter slides through the lumen, and a gripping configuration in which the plurality of deflectable arms engage the catheter and prevent the catheter from moving axially or rotationally.

In addition or alternatively, and in another example, the second member defines a lumen having a proximal region configured to receive the plurality of deflectable arms in the release configuration a middle region configured to receive the plurality of deflectable arms in the gripping configuration, and a distal region configured to receive the catheter, wherein when the plurality of deflectable arms of the actuatable clamp are moved from the proximal region into the middle region, the plurality of deflectable arms are compressed radially inward into engagement with the catheter.

In another example, a method of torqueing a catheter disposed over a guidewire comprises loading a catheter shaft attached to a catheter hub over a guidewire, loading a guidewire control device onto the guidewire, the guidewire control device including a housing having a passage for receiving the guidewire, a clutch connected to the housing and actuatable between a release configuration in which the guidewire is freely moveable within the passage and a gripping configuration in which the guidewire is prevented from rotational and axial movement relative to the housing, and an interface rotatably connected to the housing, wherein the clutch is in the release configuration, attaching the catheter hub to the interface, actuating the clutch to the gripping configuration thereby preventing rotational and axial movement of the guidewire relative to the housing, and torqueing the catheter by rotating the interface.

In addition or alternatively, and in another example, before loading the catheter shaft over the guidewire, the method further comprises loading an accessory device onto the catheter shaft, the accessory device including an actuator having a lumen configured to receive the catheter shaft, the actuator having a distal end defining an actuatable clamp having a release configuration in which the catheter shaft is freely moveable within the lumen and a gripping configuration in which the catheter shaft is prevented from rotational and axial movement relative to the accessory device, the accessory device further including a sleeve configured to actuate the actuatable clamp, wherein the actuatable clamp is in the release configuration, actuating the accessory device by moving the sleeve over the actuatable clamp thereby moving the actuatable clamp into the gripping configuration, and wherein torqueing the catheter includes rotating the interface and the accessory device in the same direction.

The above summary of some embodiments, aspects, and/or examples is not intended to describe each embodiment or every implementation of the present disclosure. The figures and the detailed description which follows more particularly exemplify these embodiments.

### Brief Description of the Drawings

The disclosure may be more completely understood in consideration of the following detailed description of various embodiments in connection with the accompanying drawings, in which:
FIG. 1 is a perspective view of an exemplary guidewire control device connected to a catheter hub;
FIG. 2 is an exploded view of the guidewire control device and catheter hub of FIG. 1;
FIG. 3A is a cross-sectional view of the guidewire control device and catheter hub of FIG. 1, with the clutch mechanism in a release configuration;
FIG. 3B is a cross-sectional view of the guidewire control device and catheter hub of FIG. 1, with the clutch mechanism in a gripping configuration;
FIG. 4 shows the guidewire control device of FIG. 1 in a release configuration and being connected to a catheter hub;
FIG. 5 shows the guidewire control device of FIG. 1 in an gripping configuration;
FIG. 6 is a perspective view of an exemplary accessory device in a release configuration;
FIG. 7 is an exploded view of the accessory device of FIG. 6;
FIG. 8 is a cross-sectional view of the accessory device of FIG. 6;
FIG. 9 is a cross-sectional view of the accessory device of FIG. 6 in a gripping configuration;
FIG. 10 illustrates an assembly of the guidewire control device of FIG. 1 and the accessory device of FIG. 6 on a catheter, each device in a release configuration; and FIG. 11 illustrates the assembly of FIG. 10 with each device in a gripping configuration.

While aspects of the disclosure are amenable to various modifications and alternative forms, specifics thereof have been shown by way of example in the drawings and will be described in detail. It should be understood, however, that the intention is not to limit aspects of the disclosure to the particular embodiments described. On the contrary, the intention is to cover all modifications falling within the scope of the appended claims.

### Detailed Description

For the following defined terms, these definitions shall be applied, unless a different definition is given in the claims or elsewhere in this specification.

All numeric values are herein assumed to be modified by the term "about," whether or not explicitly indicated. The term "about", in the context of numeric values, generally refers to a range of numbers that one of skill in the art would consider equivalent to the recited value (e.g., having the same function or result). In many instances, the term "about" may include numbers that are rounded to the nearest significant figure. Other uses of the term "about" (e.g., in a context other than numeric values) may be assumed to have their ordinary and customary definition(s), as understood from and consistent with the context of the specification, unless otherwise specified.

The recitation of numerical ranges by endpoints includes all numbers within that range, including the endpoints (e.g., 1 to 5 includes 1, 1.5, 2, 2.75, 3, 3.80, 4, and 5). Although some suitable dimensions, ranges, and/or values pertaining to various components, features and/or specifications are disclosed, one of skill in the art, incited by the present disclosure, would understand desired dimensions, ranges, and/or values may deviate from those expressly disclosed.

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise. It is to be noted that in order to facilitate understanding, certain features of the disclosure may be described in the singular, even though those features may be plural or recurring within the disclosed embodiment(s). Each instance of the features may include and/or be encompassed by the singular disclosure(s), unless expressly stated to the contrary. For simplicity and clarity purposes, not all elements of the disclosure are necessarily shown in each figure or discussed in detail below. However, it will be understood that the following discussion may apply equally to any and/or all of the components for which there are more than one, unless explicitly stated to the contrary. Additionally, not all instances of some elements or features may be shown in each figure for clarity.

Relative terms such as "proximal", "distal", "advance", "withdraw", variants thereof, and the like, may be generally considered with respect to the positioning, direction, and/or operation of various elements relative to a user/operator/manipulator of the device, wherein "proximal" and "withdraw" indicate or refer to closer to or toward the user and "distal" and "advance" indicate or refer to farther from or away from the user. In some instances, the terms "proximal" and "distal" may be arbitrarily assigned in an effort to facilitate understanding of the disclosure, and such instances will be readily apparent to the skilled artisan. Other relative terms, such as "upstream", "downstream", "inflow", and "outflow" refer to a direction of fluid flow within a lumen, such as a body lumen, a blood vessel, or within a device.

The term "extent" may be understood to mean a greatest measurement of a stated or identified dimension, unless the extent or dimension in question is preceded by or identified as a "minimum", which may be understood to mean a smallest measurement of the stated or identified dimension. For example, "outer extent" may be understood to mean a maximum outer dimension, "radial extent" may be understood to mean a maximum radial dimension, "longitudinal extent" may be understood to mean a maximum longitudinal dimension, etc. Each instance of an "extent" may be different (e.g., axial, longitudinal, lateral, radial, circumferential, etc.) and will be apparent to the skilled person from the context of the individual usage. Generally, an "extent" may be considered a greatest possible dimension measured according to the intended usage, while a "minimum extent" may be considered a smallest possible dimension measured according to the intended usage. In some instances, an "extent" may generally be measured orthogonally within a plane and/or cross-section, but may be, as will be apparent from the particular context, measured differently - such as, but not limited to, angularly, radially, circumferentially (e.g., along an arc), etc.

The terms "monolithic" and "unitary" shall generally refer to an element or elements made from or consisting of a single structure or base unit/element. A monolithic and/or unitary element shall exclude structure and/or features made by assembling or otherwise joining multiple discrete elements together.

It is noted that references in the specification to "an embodiment", "an example", "some embodiments", "some examples", "other embodiments", "other examples" etc., indicate that the embodiment(s) or example(s) described may include a particular feature, structure, or characteristic, but every embodiment or example may not necessarily include the particular feature, structure, or characteristic. Moreover, such phrases are not necessarily referring to the same embodiment or example. Further, when a particular feature, structure, or characteristic is described in connection with one embodiment or example, it would be within the knowledge of one skilled in the art to effect the particular feature, structure, or characteristic in connection with other embodiments or examples, whether or not explicitly described, unless clearly stated to the contrary. That is, the various individual elements described below, even if not explicitly shown in a particular combination, are nevertheless contemplated as being combinable or arrangeable with each other to form other additional embodiments or examples or to complement and/or enrich the described embodiment(s) or example(s), as would be understood by one of ordinary skill in the art.

For the purpose of clarity, certain identifying numerical nomenclature (e.g., first, second, third, fourth, etc.) may be used throughout the description and/or claims to name and/or differentiate between various described and/or claimed features. It is to be understood that the numerical nomenclature is not intended to be limiting and is exemplary only. In some embodiments, alterations of and deviations from previouslyused numerical nomenclature may be made in the interest of brevity and clarity. That is, a feature identified as a "first" element may later be referred to as a "second" element, a "third" element, etc. or may be omitted entirely, and/or a different feature may be referred to as the "first" element. The meaning and/or designation in each instance will be apparent to the skilled practitioner.

The following description should be read with reference to the drawings, which are not necessarily to scale, wherein similar elements in different drawings are numbered the same. The detailed description and drawings are intended to illustrate but not limit the disclosure. Those skilled in the art will recognize that the various elements described and/or shown may be arranged in various combinations and configurations without departing from the scope of the disclosure. The detailed description and drawings illustrate example embodiments of the disclosure. However, in the interest of clarity and ease of understanding, while every feature and/or element may not be shown in each drawing, the feature(s) and/or element(s) may be understood to be present regardless, unless otherwise specified.

Treating patients with diseased arteries often involves crossing an occlusion with a guidewire. A support catheter may be used to provide column support to the guidewire. During the procedure, the support catheter hub is often torqued while holding it in the user's primary hand while simultaneously clutching the guidewire with the little finger of the primary hand to avoid torqueing the guidewire. The user may then torque the catheter using a pinch grip with the second hand. This procedure has disadvantages including the difficulties of clutching a small diameter guidewire with the little finger, especially as the guidewire often has a hydrophilic coating and is usually wet. The procedure often lasts 15-20 minutes, and clutching the guidewire and/or torqueing the catheter multiple times during the procedure using the hands often results in muscle strain. The catheter shaft and hub generally have different diameters and torqueing both simultaneously may transfer differential torque due to differences in diameters, and this may result in kinking and damaging the catheter shaft. Devices for simultaneously gripping the guidewire to hold it stationary while torqueing the catheter shaft are needed.

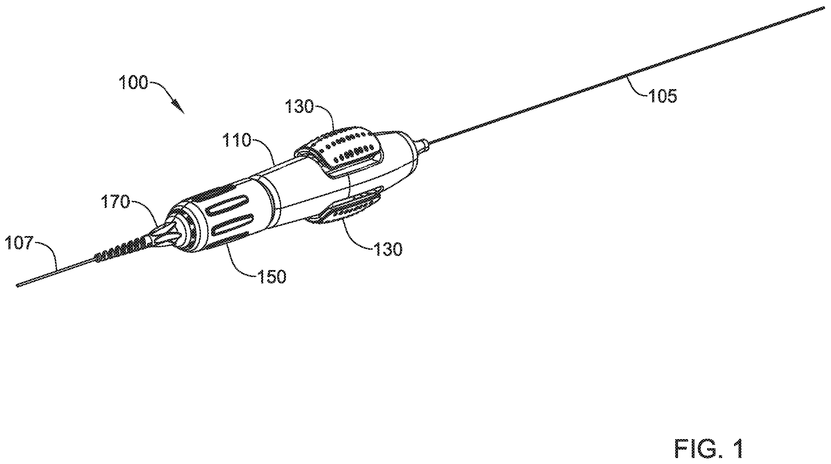

As will be described in greater detail below, FIG. 1 illustrates an example guidewire control device 100 including a housing 110, an attached clutch mechanism 130, and an interface 150 rotatably coupled to the housing 110. The housing 110 is configured to receive a guidewire 105, and the interface 150 is configured to receive a catheter hub 170 and catheter shaft 107. The interface 150 may be configured to engage the catheter hub 170 and catheter shaft 107 such that rotation of the interface 150 causes rotation of the catheter hub 170 and catheter shaft 107. The clutch mechanism 130 may be actuatable to selectively grip and release the guidewire 105. The guidewire control device 100 is configured such that when the clutch mechanism 130 grips the guidewire 105, the guidewire 105 is prevented from moving rotationally and axially, while the interface 150 and attached catheter hub 170 and catheter shaft 107 freely rotate relative to the housing 110.

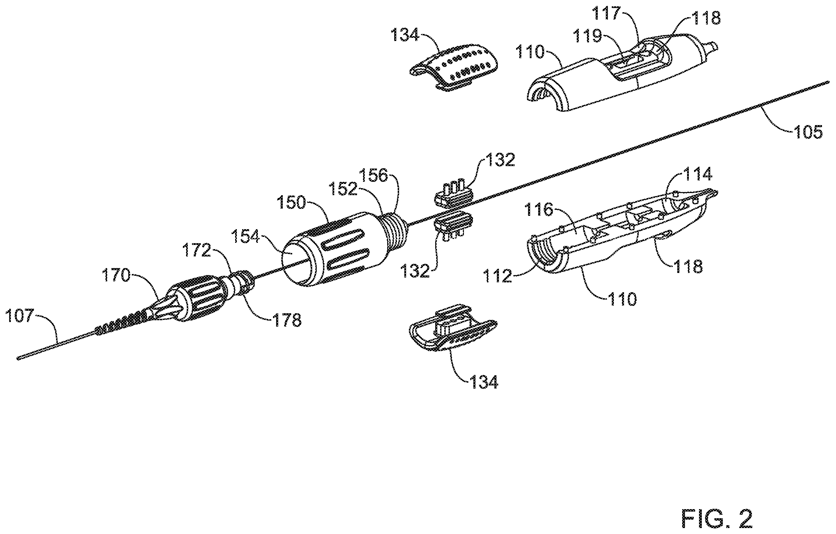

The details of the guidewire control device 100 are illustrated in FIG. 2. The housing 110 may be formed in two parts as shown, or may be formed as a single piece. When the housing 110 is formed in two parts, the parts may be connected by a friction fit, a snap fit, with adhesive, welding, or any other suitable connection. The housing 110 may define a passage 114 extending longitudinally through the housing 110 for receiving the guidewire 105. The passage 114 may be configured to allow the guidewire 105 to freely slide back and forth without obstruction.

The passage 114 may include a chamber 116 at the distal end of the housing 110. The chamber 116 may be sized and shaped to receive the proximal region 156 of the interface 150 in a rotatable connection such that the interface 150 may freely rotate relative to the housing 110. The inner surface of the chamber 116 may have one or more channel 112 configured to receive one or more protrusion 152 on the outer surface of the proximal region 156 of the interface 150. In other examples, the inner surface of the chamber 116 may have the one or more protrusion and the outer surface of the proximal region 156 of the interface 150 may have the one or more channel. In the illustrated example, the chamber 116 has a plurality of circumferential channels 112 and the protrusions 152 on the proximal region 156 of the interface 150 include a plurality of circumferential ridges 152 configured to rotate within the channels 112. In other examples, the protrusion 152 may be one or more pin, peg, or bump configured to slide within the channel 112. Alternatively, the proximal region 156 of the interface 150 may engage the inner surface of the chamber 116 with a snap fit that allows for free rotation of the interface 150 relative to the housing 110.

The housing 110 may have a pair of recesses 118 in opposing sides. The recesses 118 may be configured to receive the clutch mechanism 130, which may include a pair of clutch grips 134 connected to a pair of engagement members 132. Each recess 118 may have a first portion 117 configured to receive the clutch grip 134, and a second portion 119 extending into the passage 114 and configured to receive the engagement member 132.

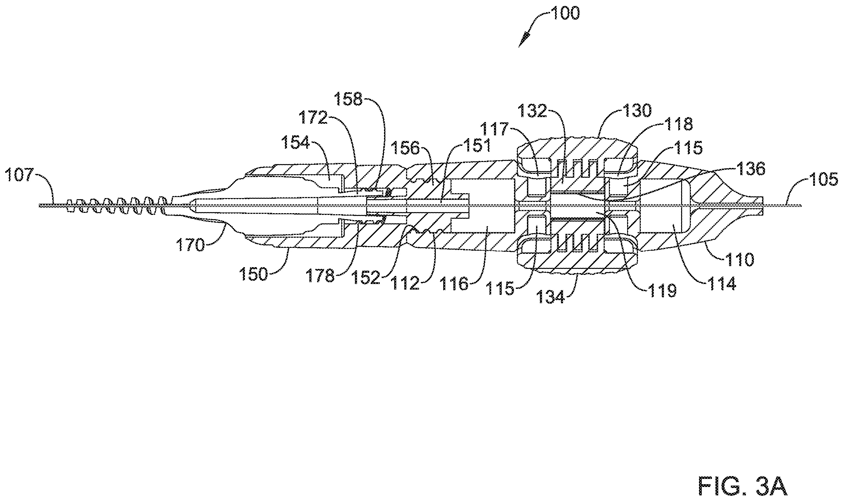

The interface 150 may have a cavity 154 in the distal end configured to receive the proximal end 172 of a catheter hub 170. The catheter hub 170 may be a conventional hub attached to a catheter shaft 107. The proximal end 172 of the catheter hub 170 may have external threading 178 configured to engage internal threading 158 on the inner surface of the cavity 154 of the interface 150, as shown in FIG. 3A. The proximal region 156 of the interface 150 may define a lumen 151 configured for receiving the guidewire 105 such that the guidewire 105 may extend through the housing 110, interface 150, and catheter hub 170. The threading engagement between the interface 150 and catheter hub 170 results in the two parts being rotated together. Rotation of the interface 150 causes equal rotation of the catheter hub 170 and catheter shaft 107. The rotatable connection between the interface 150 and housing 110 allows the interface 150 to be rotated while the housing 110 remains stationary.

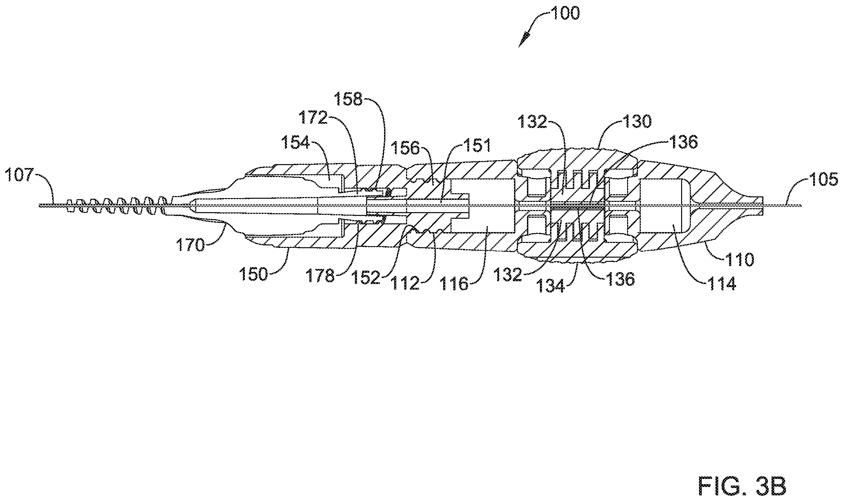

The clutch mechanism 130 is actuatable between a release configuration, shown in FIG. 3A, to a gripping configuration, shown in FIG. 3B. In the release configuration shown in FIG. 3A, the clutch grips 134 extend radially outward from the outer surface of the housing 110 and the engagement members 132 are spaced away from the guidewire 105, allowing the guidewire 105 to freely move rotationally and axially within the passage 114 of the housing 110. The clutch grips 134 may be biased in the release configuration. In some examples, a spring element (not shown) may be disposed within the recess 118, holding the clutch grips 134 and the connected engagement members 132 in the release configuration. In one example, a spring element (not shown) may be disposed within cavities 115 in the housing 110 on one or both sides of the engagement members 132, biasing the clutch grips 134 in the release configuration.

Actuating the clutch mechanism 130 may involve pressing down on the clutch grips 134 which moves the engagement members 132 into contact with the guidewire 105, as shown in FIG. 3B. In some examples, the engagement members 132 may be pads with an engagement surface 136 facing the guidewire 105. In some examples, two opposing engagement members 132 may be pressed against one another, capturing the guidewire 105 between engagement surfaces 136 and preventing any rotational or axial movement of the guidewire 105 relative to the housing 110. Actuation of the clutch mechanism 130 may be achieved by gripping the housing 110 in one hand, and squeezing the opposing clutch grips 134 between the fingers and palm. In some examples, the user may squeeze the clutch grips 134 with the ring and/or middle finger and palm, leaving the thumb and first finger free to rotate the interface 150, as will be described below. When the user releases the clutch grips 134, they move back to the release configuration, allowing the guidewire 105 to move freely within the passage 114 of the housing.

In some examples, the engagement surfaces 136 may be textured such that squeezing the engagement surfaces 136 together against the guidewire 105 prevents rotation or axial movement of the guidewire 105, without damaging the guidewire 105. In other examples, the engagement surfaces 136 may be made of a polymer such as silicone. In some examples, the engagement surfaces 136 may include a material that provides an enhanced gripping surface for holding a hydrophilic and/or wet guidewire 105. Examples of such materials include elastomers or tacky materials such as a tacky silicone.

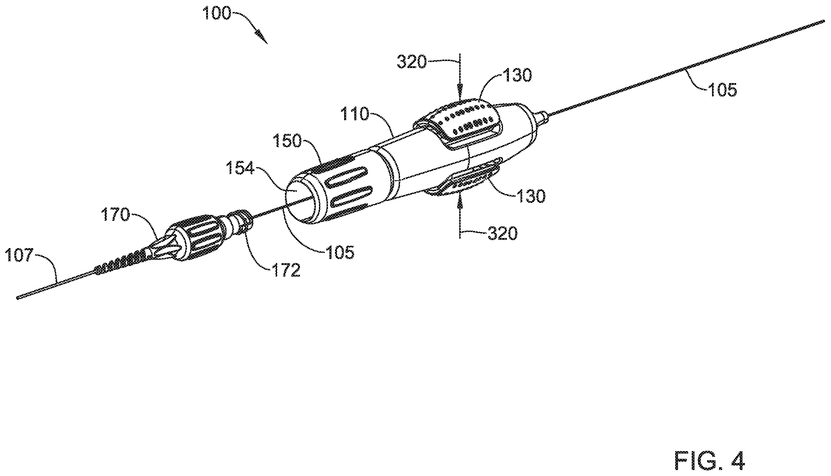

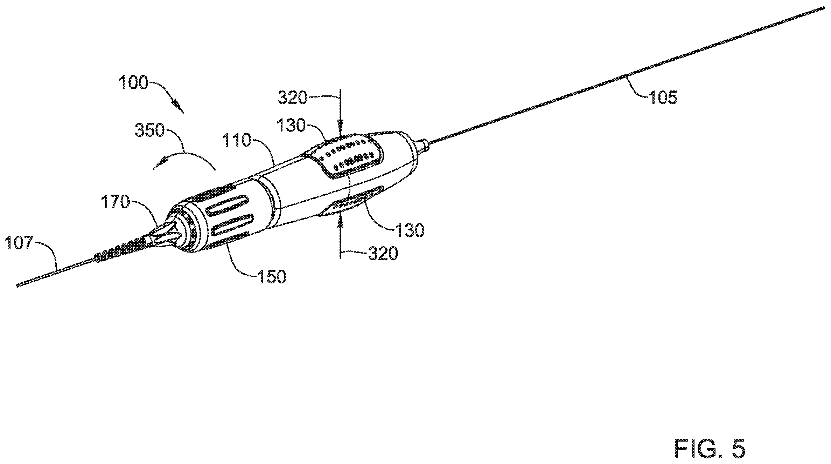

In operation, the user may first load a support catheter shaft 107 attached to a catheter hub 170 over a guidewire 105. Next, the guidewire control device 100, with the clutch mechanism 130 in the release configuration, may be loaded over the guidewire 105 from the proximal end of the guidewire 105, as shown in FIG. 4. The guidewire control device 100 may then be advanced over the catheter hub 170, with the proximal end 172 inserted into the cavity 154 of the interface 150. The interface 150 may then be attached to the catheter hub 170 by threading the interface 150 onto the proximal end 172 of the catheter hub 170 by rotating the interface 150. The clutch mechanism 130 may then actuated to grip the guidewire 105 by applying force in the direction of arrows 320, as shown in FIGS. 4 and 5. With the guidewire control device 100 gripping the guidewire 105, the interface 150 may be rotated as indicated by arrow 350 in FIG. 5, to torque the catheter shaft 107. In some examples, in order to maintain the clutch mechanism 130 in the gripping configuration, shown in FIG. 3B, the force applied in the direction of arrows 320 must be maintained, generally by the user holding the housing 110. When the user releases the clutch mechanism 130, it returns to the release configuration, shown in FIG. 3A. In other examples, pressing the clutch mechanism once may grip the guidewire, and pressing the clutch mechanism a second time may release the guidewire. In still other examples, a release mechanism may be provided to move the clutch mechanism from the gripping configuration to the release configuration.

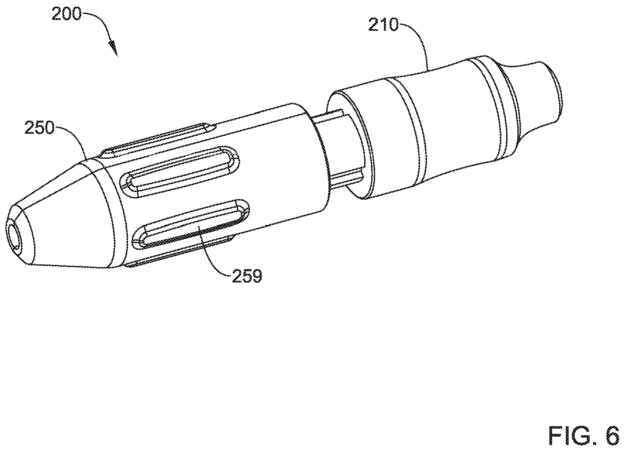

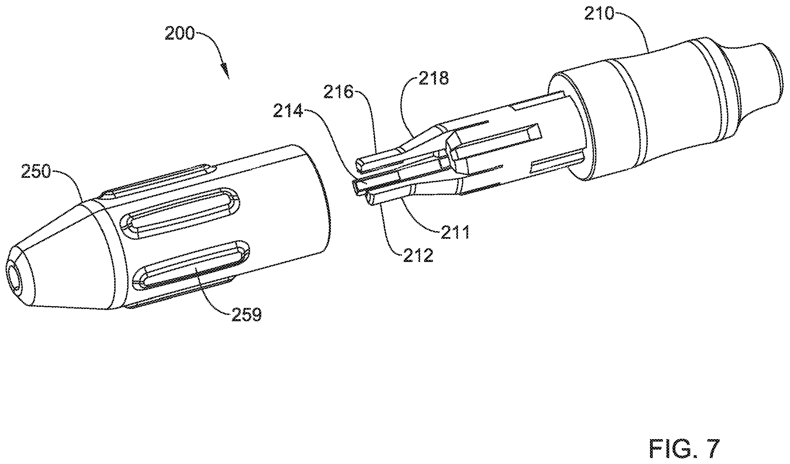

An accessory device 200, as shown in FIGS. 6-9, may be used in combination with the guidewire control device 100 to provide additional torqueing of the catheter shaft. The accessory device 200 may include an actuator 210 and a sleeve 250. The sleeve 250 may include one or more protrusions such as ridges 259 shown in FIG. 6. The ridges 259 may provide an enhanced gripping surface to the sleeve 250. As shown in FIG. 7, the distal end of the actuator 210 may include a collet type structure including an actuatable clamp 211 configured to receive a catheter shaft. In some examples, the actuatable clamp 211 may include a plurality of deflectable arms 212 surrounding a lumen 214. Each deflectable arm 212 may include a distal region 216 substantially aligned with a longitudinal axis of the actuator 210, and an angled region 218 proximal of the distal region 216. In the example shown in FIG. 7, the actuator 210 has three deflectable arms 212, however it will be understood that the actuator 210 may include two or more than three deflectable arms 212. The sleeve 250 may be configured to slide over the actuatable clamp 211 to move the deflectable arms 212 inward toward the lumen 214, into a gripping configuration around the catheter shaft. The plurality of deflectable arms 212 may be moveable between a release configuration in which the catheter slides freely through the lumen 214, and a gripping configuration in which the plurality of deflectable arms 212 engage and grip the catheter shaft and prevent the catheter shaft from moving axially or rotationally relative to the accessory device 200. When in the gripping configuration, rotation of the actuator 210 or the sleeve 250 results in simultaneous and equal rotation of the catheter shaft held within the accessory device 200.

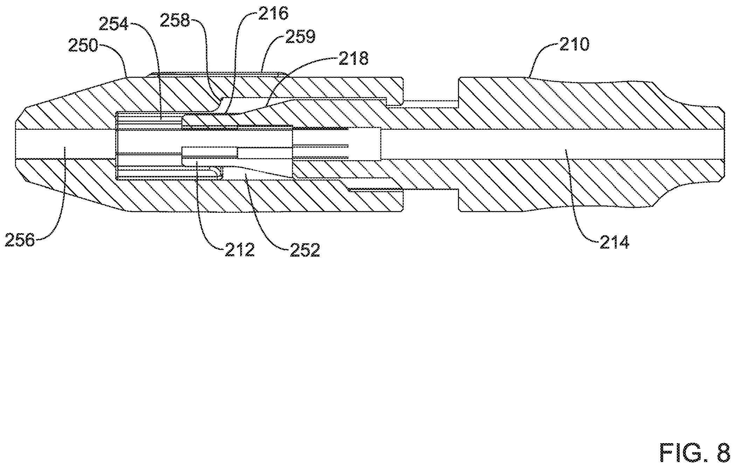

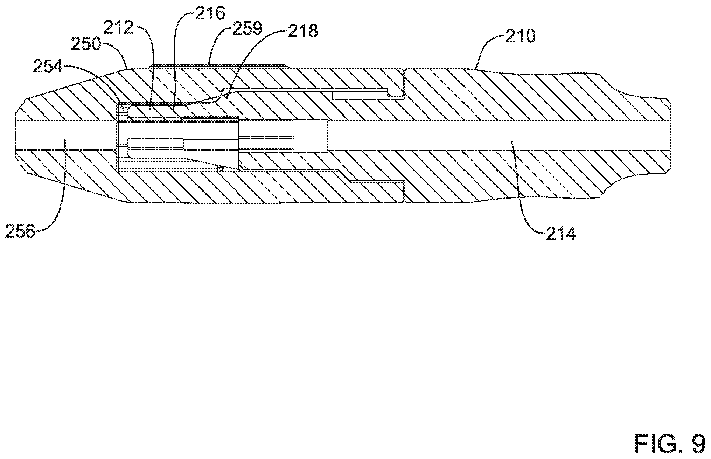

The cross-sectional views in FIGS. 8 and 9 illustrate the details of the actuator 210 and sleeve 250. The sleeve 250 may have a proximal chamber 252 configured to receive the deflectable arms 212 in an expanded or release configuration, and a central chamber 254 configured to receive the deflectable arms 212 in a contracted or gripping configuration. The sleeve 250 may define a lumen 256 extending from a distal end of the sleeve 250 through the central chamber 254 and proximal chamber 252 such that when the sleeve 250 is disposed over the actuator 210, the lumen 256, central chamber 254, proximal chamber 252, and lumen 214 through the actuator 210 define a pathway for receiving a catheter shaft (not shown). In some examples, the inner surfaces of the deflectable arms 212 and/or the lumen 214 of the actuator 210 may have an elastomeric lining (not shown) to aid in gripping the catheter shaft. The proximal chamber 252 may have a first inner diameter and the central chamber 254 may have a second inner diameter. As shown in FIG. 8, the first inner diameter of the proximal chamber 252 may be larger than the second inner diameter of the central chamber 254. A shoulder 258 may be formed at the transition between the central and proximal chambers. The sleeve 250 may be configured to slide over the deflectable arms 212 of the actuator 210. As the distal region 216 of each deflectable arm 212 moves from the proximal chamber 252 into the central chamber 254, the angled region 218 of the deflectable arm 212 engages the shoulder 258, which pushes the angled region 218 of the deflectable arm 212 radially inward, compressing the arms 212 as shown in FIG. 9, clamping a catheter shaft disposed within the lumen 214 of the actuator 210. The catheter shaft is not shown in FIGS. 8 and 9 so as not to obscure the details of the actuator 210 and sleeve 250. However, when present, the catheter shaft would extend through the lumen 256 and central chamber 254 of the sleeve 250, and the lumen 214 of the actuator 210.

The actuator 210 may have the same outer diameter as the sleeve 250, such that when coupled around a catheter shaft, the accessory device 200 provides a uniform outer diameter for gripping and torqueing, as shown in FIG. 9. In some examples, one or both of the sleeve 250 and actuator 210 may include one or more protrusions such as the ridges 259 shown on the sleeve 250 in FIG. 6, for aiding the user in gripping and torqueing the accessory device 200. In some examples, the accessory device 200 may have an outer diameter the same as or similar to the outer diameter of the catheter hub 170 or interface 150 to enable a user to hold the guidewire control device 100 in one hand and the accessory device 200 in the other hand and apply equal torque on the catheter shaft with both hands when the accessory device 200 is placed on the same catheter shaft as the guidewire control device 100.

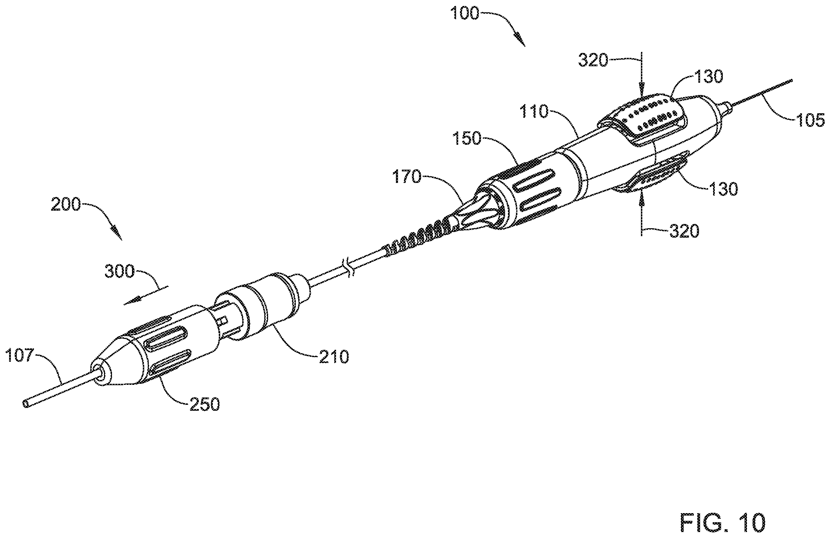

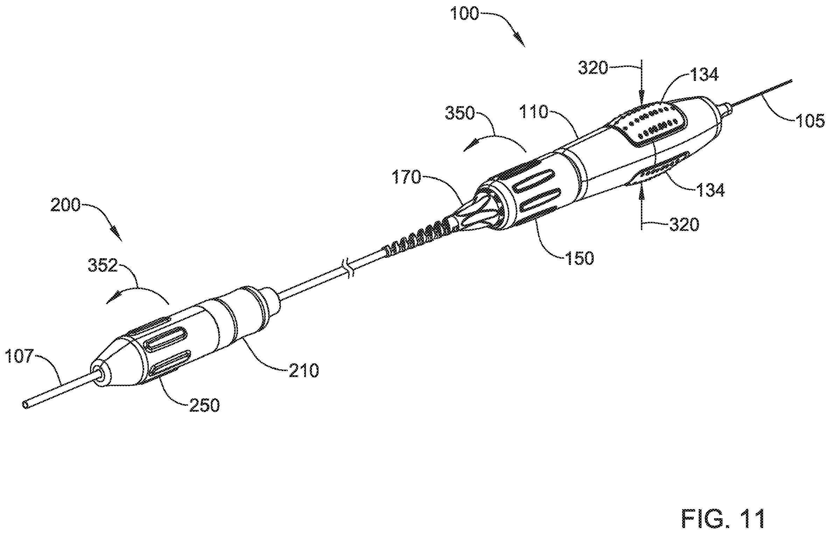

FIGS. 10 and 11 illustrate the guidewire control device 100 and accessory device 200 being attached to the same catheter shaft 107 to allow the user to torque the catheter shaft 107 using both hands while easily holding the guidewire 105 stationary. The accessory device 200 may be loaded onto the catheter shaft 107 while in the release configuration. The accessory device 200 may be loaded onto the catheter shaft 107 with attached catheter hub 170 from the distal end of the catheter shaft 107 before loading the catheter shaft 107 onto the guidewire 105. The accessory device 200 may be actuated to the gripping configuration before or after the guidewire control device 100 is attached. Once the catheter shaft 107, catheter hub 170, and accessory device 200 have been loaded onto the guidewire 105, the guidewire control device 100 may be loaded onto the guidewire 105 from the proximal end of the guidewire 105, and threaded onto the catheter hub 170, as discussed above, with the clutch mechanism 130 in the release configuration. Upon inserting the catheter shaft 107 into the patient, the clutch mechanism 130 may be actuated by applying force in the direction of arrows 320, as discussed above, to grip the guidewire 105 and prevent rotational and axial movement of the guidewire 105 relative to the guidewire control device 100. If the accessory device 200 was not previously actuated to the gripping configuration, the user may then close the gap between the actuator 210 and the sleeve 250 of the accessory device 200 by moving the actuator 210 into the sleeve 250 as indicated by arrow 300. This will actuate the collet design of the deflectable arms 212 to grip the catheter shaft 107 in the gripping configuration.

As shown in FIG. 11, the catheter hub 170 and attached catheter shaft 107 may be torqued by rotating the interface 150 as indicated by arrow 350, and the catheter shaft 107 may be torqued by rotating the accessory device 200 as indicated by arrow 352. The user may grip the housing 110 in the primary hand, holding the clutch grips 134 in the gripping configuration by applying pressure as indicated by arrows 320, to hold the guidewire 105 stationary while the catheter shaft 107 is torqued by rotating the interface 150, such as with the thumb and first finger. The opposite hand may be used to grip and rotate the accessory device 200. The combination of the guidewire control device 100 and accessory device 200 provide the user with improved catheter shaft 107 torqueing ability while preventing rotational and axial movement of the guidewire 105. The outer diameters of the interface 150 and accessory device 200 may be the same to minimize differential torque transmission by the two devices. If the guidewire control device 100 needs to be moved relative to the guidewire 105, the user may release the clutch grips 134, allowing the clutch grips 134 to move to the biased, release configuration.

In some examples, the guidewire control device 100 and the accessory device 200 may be made of polymer, elastomeric polyamides, block polyamide/ethers, polyether block amide (PEBA, for example available under the trade name PEBAX^{®}), ethylene tetrafluoroethylene (ETFE), or other polymers generally used in medical catheters.

The materials that can be used for the various components of the guidewire control device 100 and the accessory device 200 (and/or other systems or components disclosed herein) and the various elements thereof disclosed herein may include those commonly associated with medical devices. For simplicity purposes, the following discussion makes reference to the guidewire control device 100 and the accessory device 200 (and variations, systems or components disclosed herein). However, this is not intended to limit the devices and methods described herein, as the discussion may be applied to other elements, members, components, or devices disclosed herein.

In some embodiments, the guidewire control device 100 and the accessory device 200 (and variations, systems or components thereof disclosed herein) may be made from a metal, metal alloy, ceramics, zirconia, polymer (some examples of which are disclosed below), a metal-polymer composite, combinations thereof, and the like, or other suitable material. Some examples of suitable metals and metal alloys include stainless steel, such as 444V, 444L, and 314LV stainless steel; mild steel; nickel-titanium alloy such as linear-elastic and/or super-elastic nitinol; cobalt chromium alloys, titanium and its alloys, alumina, metals with diamond-like coatings (DLC) or titanium nitride coatings, other nickel alloys such as nickel-chromium-molybdenum alloys (e.g., UNS: N06625 such as INCONEL^{®} 625, UNS: N06022 such as HASTELLOY^{®} C-22^{®}, UNS: N10276 such as HASTELLOY^{®} C276^{®}, other HASTELLOY^{®} alloys, and the like), nickel-copper alloys (e.g., UNS: N04400 such as MONEL^{®} 400, NICKELVAC^{®} 400, NICORROS^{®} 400, and the like), nickel-cobalt-chromium-molybdenum alloys (e.g., UNS: R44035 such as MP35-N^{®} and the like), nickel-molybdenum alloys (e.g., UNS: N10665 such as HASTELLOY^{®} ALLOY B2^{®}), other nickel-chromium alloys, other nickel-molybdenum alloys, other nickelcobalt alloys, other nickel-iron alloys, other nickel-copper alloys, other nickel-tungsten or tungsten alloys, and the like; cobalt-chromium alloys; cobalt-chromium-molybdenum alloys (e.g., UNS: R44003 such as ELGILOY^{®}, PHYNOX^{®}, and the like); platinum enriched stainless steel; titanium; platinum; palladium; gold; combinations thereof; and the like; or any other suitable material.

As alluded to herein, within the family of commercially available nickel-titanium or nitinol alloys, is a category designated "linear elastic" or "non-super-elastic" which, although may be similar in chemistry to conventional shape memory and super elastic varieties, may exhibit distinct and useful mechanical properties. Linear elastic and/or non-super-elastic nitinol may be distinguished from super elastic nitinol in that the linear elastic and/or non-super-elastic nitinol does not display a substantial "superelastic plateau" or "flag region" in its stress/strain curve like super elastic nitinol does. Instead, in the linear elastic and/or non-super-elastic nitinol, as recoverable strain increases, the stress continues to increase in a substantially linear, or a somewhat, but not necessarily entirely linear relationship until plastic deformation begins or at least in a relationship that is more linear than the super elastic plateau and/or flag region that may be seen with super elastic nitinol. Thus, for the purposes of this disclosure linear elastic and/or non-super-elastic nitinol may also be termed "substantially" linear elastic and/or non-super-elastic nitinol.

In some cases, linear elastic and/or non-super-elastic nitinol may also be distinguishable from super elastic nitinol in that linear elastic and/or non-super-elastic nitinol may accept up to about 2-5% strain while remaining substantially elastic (e.g., before plastically deforming) whereas super elastic nitinol may accept up to about 8% strain before plastically deforming. Both of these materials can be distinguished from other linear elastic materials such as stainless steel (that can also be distinguished based on its composition), which may accept only about 0.2 to 0.44 percent strain before plastically deforming.

In some embodiments, the linear elastic and/or non-super-elastic nickel-titanium alloy is an alloy that does not show any martensite/austenite phase changes that are detectable by differential scanning calorimetry (DSC) and dynamic metal thermal analysis (DMTA) analysis over a large temperature range. For example, in some embodiments, there may be no martensite/austenite phase changes detectable by DSC and DMTA analysis in the range of about -60 degrees Celsius (°C) to about 120 °C in the linear elastic and/or non-super-elastic nickel-titanium alloy. The mechanical bending properties of such material may therefore be generally inert to the effect of temperature over this very broad range of temperature. In some embodiments, the mechanical bending properties of the linear elastic and/or non-super-elastic nickel-titanium alloy at ambient or room temperature are substantially the same as the mechanical properties at body temperature, for example, in that they do not display a super-elastic plateau and/or flag region. For example, across a broad temperature range, the linear elastic and/or non-super-elastic nickel-titanium alloy maintains its linear elastic and/or non-super-elastic characteristics and/or properties.

In some embodiments, the linear elastic and/or non-super-elastic nickel-titanium alloy may be in the range of about 50 to about 60 weight percent nickel, with the remainder being essentially titanium. In some embodiments, the composition is in the range of about 54 to about 57 weight percent nickel. One example of a suitable nickel-titanium alloy is FHP-NT alloy commercially available from Furukawa Techno Material Co. of Kanagawa, Japan. Other suitable materials may include ULTANIUM^{™} (available from Neo-Metrics) and GUM METAL^{™} (available from Toyota). In some other embodiments, a superelastic alloy, for example a superelastic nitinol can be used to achieve desired properties.

In some embodiments, the guidewire control device 100 and the accessory device 200 (and variations, systems or components thereof disclosed herein) and/or portions thereof, may be made from or include a polymer or other suitable material. Some examples of suitable polymers may include polytetrafluoroethylene (PTFE), ethylene tetrafluoroethylene (ETFE), fluorinated ethylene propylene (FEP), polyoxymethylene (POM, for example, DELRIN^{®} available from DuPont), polyether block ester, polyurethane (for example, Polyurethane 85A), polypropylene (PP), polyvinylchloride (PVC), polyether-ester (for example, ARNITEL^{®} available from DSM Engineering Plastics), ether or ester based copolymers (for example, butylene/poly(alkylene ether) phthalate and/or other polyester elastomers such as HYTREL^{®} available from DuPont), polyamide (for example, DURETHAN^{®} available from Bayer or CRISTAMID^{®} available from Elf Atochem), elastomeric polyamides, block polyamide/ethers, polyether block amide (PEBA, for example available under the trade name PEBAX^{®}), ethylene vinyl acetate copolymers (EVA), silicones, polyethylene (PE), Marlex high-density polyethylene, Marlex low-density polyethylene, linear low density polyethylene (for example REXELL^{®}), polyester, polybutylene terephthalate (PBT), polyethylene terephthalate (PET), polytrimethylene terephthalate, polyethylene naphthalate (PEN), polyetheretherketone (PEEK), polyimide (PI), polyetherimide (PEI), polyphenylene sulfide (PPS), polyphenylene oxide (PPO), poly paraphenylene terephthalamide (for example, KEVLAR^{®}), polysulfone, nylon, nylon-12 (such as GRILAMID^{®} available from EMS American Grilon), perfluoro(propyl vinyl ether) (PFA), ethylene vinyl alcohol, polyolefin, polystyrene, epoxy, polyvinylidene chloride (PVdC), poly(styrene-*b*-isobutylene-*b-*styrene) (for example, SIBS and/or SIBS 50A), polycarbonates, ionomers, polyurethane silicone copolymers (for example, ElastEon^{®} from Aortech Biomaterials or ChronoSil^{®} from AdvanSource Biomaterials), biocompatible polymers, other suitable materials, or mixtures, combinations, copolymers thereof, polymer/metal composites, and the like. In some embodiments, the sheath can be blended with a liquid crystal polymer (LCP). For example, the mixture can contain up to about 6 percent LCP.

It should be understood that this disclosure is, in many respects, only illustrative. Changes may be made in details, particularly in matters of shape, size, and arrangement of steps without exceeding the scope of the disclosure. This may include, to the extent that it is appropriate, the use of any of the features of one example embodiment being used in other embodiments. The disclosure's scope is, of course, defined in the language in which the appended claims are expressed.

## Claims

1. A guidewire control device (100) comprising:
a housing (110) having a passage (114) for receiving a guidewire (105);
a clutch (130) including first and second opposing grips (134) biased in a release configuration, the clutch (130) being connected to the housing (110) and actuatable to selectively grip and release the guidewire (105) disposed in the passage (114); and
an interface (150) rotatably connected to the housing (110), the interface (150) configured to receive a catheter hub (170) and torque the catheter hub (170).

2. The guidewire control device (100) of claim 1, wherein the clutch (130) incudes an engagement member (132) configured to grip the guidewire (105) and prevent rotation of the guidewire (105) while the interface (150) rotates.

3. The guidewire control device (100) of claim 2, wherein the engagement member (132) includes first and second opposing pads (132) configured to be moved toward each other and into contact with the guidewire (105).

4. The guidewire control device (100) of claim 3, wherein the first and second opposing grips (134) are attached to the first and second opposing pads (132), respectively, the first and second opposing grips (134) configured to move between the release configuration and a gripping configuration, wherein when in the release configuration, the first and second opposing grips (134) extend radially outward from an outer surface of the housing (110), and when in the gripping configuration, the first and second opposing grips (134) are disposed radially inward toward the passage (114).

5. The guidewire control device (100) of claim 1, wherein the interface (150) includes a proximal end (156) defining a lumen (151) for receiving the guidewire (105), the proximal end (156) having at least one protrusion (152) configured to rotate within at least one channel (112) on an inner surface of the passage (114) of the housing (110).

6. The guidewire control device (100) of claim 5, wherein the interface (150) includes a distal end defining a cavity (154) having internal threading (158) configured to receive an externally threaded proximal end (156) of the catheter hub (170).

7. The guidewire control device (100) of any one of claims 1-6, further comprising an accessory device (200) including an actuator (210) having a lumen (214) configured to receive a catheter (107), the actuator (210) having a distal end defining an actuatable clamp (211) configured to engage the catheter (107), the accessory device (200) further including a sleeve (250) configured to actuate the actuatable clamp (211).

8. The guidewire control device (100) of claim 7, wherein the actuatable clamp (211) includes a plurality of deflectable arms (212) moveable between a release configuration in which the catheter (107) slides through the lumen (214), and a gripping configuration in which the plurality of deflectable arms (212) engage the catheter (107) and prevent the catheter (107) from moving axially or rotationally.

9. The guidewire control device (100) of claim 8, wherein the sleeve (250) defines a proximal chamber (252) configured to receive the plurality of deflectable arms (212) in the release configuration, a central chamber (254) configured to receive the plurality of deflectable arms (212) in the gripping configuration, and a lumen (256) configured to receive the catheter (107), wherein when the plurality of deflectable arms (212) of the actuator (210) are moved from the proximal chamber (252) into the central chamber (254), the plurality of deflectable arms (212) are compressed radially inward into engagement with the catheter (107).

10. A guidewire control assembly (100, 200) comprising:
the guidewire control device of claim 1; and
a catheter clamping device (200) including a first member (210) having a lumen (214) configured to receive a catheter (107) and a distal end defining an actuatable clamp (211) configured to engage the catheter (107), the catheter clamping device (200) further including a second member (250) having a cavity (252, 254) configured to receive and actuate the actuatable clamp (211).

11. The guidewire control assembly (100, 200) of claim 10, wherein the clutch (130) incudes an engagement member (132) configured to grip the guidewire (105) and prevent rotation of the guidewire (105) while the distal housing (150) rotates, the engagement member (132) including first and second opposing pads (132) configured to be moved toward each other and into contact with the guidewire (105).

12. The guidewire control assembly (100, 200) of claim 11, wherein the clutch (130) further includes the first and second opposing grips (134) attached to the first and second opposing pads (132), respectively, the first and second opposing grips (134) configured to move between the release configuration and a gripping configuration, wherein when in the release configuration, the first and second opposing grips (134) extend radially outward from an outer surface of the proximal housing (110), and when in the gripping configuration, the first and second opposing grips (134) are disposed radially inward toward the passage (114).

## Patentansprüche

1. Führungsdrahtsteuervorrichtung (100), aufweisend:
ein Gehäuse (110) mit einem Durchgang (114) zum Aufnehmen eines Führungsdrahts (105);
eine Kupplung (130), die einen ersten und zweiten gegenüberliegenden Griff (134) aufweist, die in eine Freigabekonfiguration vorgespannt sind, wobei die Kupplung (130) mit dem Gehäuse (110) verbunden und betätigbar ist, um den im Durchgang (114) angeordneten Führungsdraht (105) wahlweise zu ergreifen und freizugeben; und
eine Schnittstelle (150), die mit dem Gehäuse (110) drehbar verbunden ist, wobei die Schnittstelle (150) zum Aufnehmen einer Katheternabe (170) und zum Drehen der Katheternabe (170) konfiguriert ist.

2. Führungsdrahtsteuervorrichtung (100) nach Anspruch 1, wobei die Kupplung (130) ein Eingriffselement (132) aufweist, das zum Ergreifen des Führungsdrahts (105) und zum Verhindern einer Drehung des Führungsdrahts (105), während sich die Schnittstelle (150) dreht, konfiguriert ist.

3. Führungsdrahtsteuervorrichtung (100) nach Anspruch 2, wobei das Eingriffselement (132) ein erstes und zweites gegenüberliegendes Polster (132) aufweist, die dazu konfiguriert sind, aufeinander zu und in Kontakt mit dem Führungsdraht (105) bewegt werden zu können.

4. Führungsdrahtsteuervorrichtung (100) nach Anspruch 3, wobei der erste und zweite gegenüberliegende Griff (134) jeweils an dem ersten und zweiten gegenüberliegenden Polster (132) angebracht sind, wobei der erste und zweite gegenüberliegende Griff (134) dazu konfiguriert sind, sich zwischen der Freigabekonfiguration und einer Greifkonfiguration zu bewegen, wobei sich der erste und zweite gegenüberliegende Griff (134) von einer Außenfläche des Gehäuses (110) radial nach außen erstrecken, wenn sie sich in der Freigabekonfiguration befinden, und wobei der erste und zweite gegenüberliegende Griff (134) zum Durchgang (114) hin radial nach innen angeordnet sind, wenn sie sich in der Greifkonfiguration befinden.

5. Führungsdrahtsteuervorrichtung (100) nach Anspruch 1, wobei die Schnittstelle (150) ein proximales Ende (156) aufweist, das ein Lumen (151) zum Aufnehmen des Führungsdrahts (105) definiert, wobei das proximale Ende (156) mindestens einen Vorsprung (152) aufweist, der dazu konfiguriert ist, sich innerhalb mindestens eines Kanals (112) auf einer Innenfläche des Durchgangs (114) des Gehäuses (110) zu drehen.

6. Führungsdrahtsteuervorrichtung (100) nach Anspruch 5, wobei die Schnittstelle (150) ein distales Ende aufweist, das einen Hohlraum (154) mit einem Innengewinde (158) definiert, das zum Aufnehmen eines mit einem Außengewinde versehenen proximalen Endes (156) der Katheternabe (170) konfiguriert ist.

7. Führungsdrahtsteuervorrichtung (100) nach einem der Ansprüche 1 - 6, ferner aufweisend eine Zubehörvorrichtung (200), die einen Aktuator (210) mit einem Lumen (214) aufweist, das zum Aufnehmen eines Katheters (107) konfiguriert ist, wobei der Aktuator (210) ein distales Ende aufweist, das eine betätigbare Klemme (211) definiert, die dazu konfiguriert ist, den Katheter (107) in Eingriff zu nehmen, wobei die Zubehörvorrichtung (200) ferner eine Hülse (250) aufweist, die zum Betätigen der betätigbaren Klemme (211) konfiguriert ist.

8. Führungsdrahtsteuervorrichtung (100) nach Anspruch 7, wobei die betätigbare Klemme (211) mehrere auslenkbare Arme (212) aufweist, die zwischen einer Freigabekonfiguration, in der der Katheter (107) durch das Lumen (214) gleitet, und einer Greifkonfiguration, in der die mehreren auslenkbaren Arme (212) den Katheter (107) in Eingriff nehmen und den Katheter (107) an einer Axial- oder Drehbewegung hindern, bewegbar sind.

9. Führungsdrahtsteuervorrichtung (100) nach Anspruch 8, wobei die Hülse (250) eine proximale Kammer (252), die in der Freigabekonfiguration zum Aufnehmen der mehreren auslenkbaren Arme (212) konfiguriert ist, eine zentrale Kammer (254), die in der Greifkonfiguration zum Aufnehmen der mehreren auslenkbaren Arme (212) konfiguriert ist, und ein Lumen (256), das zum Aufnehmen des Katheters (107) konfiguriert ist, definiert, wobei die mehreren ablenkbaren Arme (212) in Eingriff mit dem Katheter (107) radial nach innen zusammengedrückt werden, wenn die mehreren ablenkbaren Arme (212) des Aktuators (210) von der proximalen Kammer (252) in die zentrale Kammer (254) bewegt werden.

10. Führungsdrahtsteueranordnung (100, 200), aufweisend:
die Führungsdrahtsteuervorrichtung nach Anspruch 1; und
eine Katheterklemmvorrichtung (200), die ein erstes Element (210) mit einem Lumen (214), das zum Aufnehmen eines Katheters (107) konfiguriert ist, und einem distalen Ende, das eine betätigbare Klemme (211) definiert, die dazu konfiguriert ist, den Katheter (107) in Eingriff zu nehmen, aufweist, wobei die Katheterklemmvorrichtung (200) ferner ein zweites Element (250) mit einem Hohlraum (252, 254) aufweist, der zum Aufnehmen und Betätigen der betätigbaren Klemme (211) konfiguriert ist.

11. Führungsdrahtsteueranordnung (100, 200) nach Anspruch 10, wobei die Kupplung (130) ein Eingriffselement (132) aufweist, das zum Ergreifen des Führungsdrahts (105) und zum Verhindern einer Drehung des Führungsdrahtes (105) konfiguriert ist, während sich das distale Gehäuse (150) dreht, wobei das Eingriffselement (132) ein erstes und zweites gegenüberliegendes Polster (132) aufweist, die dazu konfiguriert sind, aufeinander zu und in Kontakt mit dem Führungsdraht (105) bewegt zu werden.

12. Führungsdrahtsteueranordnung (100, 200) nach Anspruch 11, wobei die Kupplung (130) ferner den ersten und zweiten gegenüberliegenden Griff (134) aufweist, die jeweils an dem ersten und zweiten gegenüberliegenden Polster (132) angebracht sind, wobei der erste und zweite gegenüberliegende Griff (134) dazu konfiguriert sind, sich zwischen der Freigabekonfiguration und einer Greifkonfiguration zu bewegen, wobei sich der erste und zweite gegenüberliegende Griff (134) von einer Außenfläche des proximalen Gehäuses (110) radial nach außen erstrecken, wenn sie sich in der Freigabekonfiguration befinden, und wobei der erste und zweite gegenüberliegende Griff (134) in Richtung des Durchgangs (114) radial nach innen angeordnet sind, wenn sie sich in der Greifkonfiguration befinden.

## Revendications

1. Dispositif de commande de fil-guide (100) comprenant :
un boîtier (110) ayant un passage (114) pour recevoir un fil-guide (105) ;
un embrayage (130) comprenant des premier et deuxième dispositifs de préhension (134) opposés sollicités dans une configuration de libération, l'embrayage (130) étant raccordé au boîtier (110) et pouvant être actionné pour saisir et libérer sélectivement le fil-guide (105) disposé dans le passage (114) ; et
une interface (150) raccordée, en rotation, au boîtier (110), l'interface (150) étant configurée pour recevoir un raccord de cathéter (170) et pour serrer le raccord de cathéter (170).

2. Dispositif de commande de fil-guide (100) selon la revendication 1, dans lequel l'embrayage (130) comprend un élément de mise en prise (132) configuré pour saisir le fil-guide (105) et pour empêcher la rotation du fil-guide (105) lors de la rotation de l'interface (150).

3. Dispositif de commande de fil-guide (100) selon la revendication 2, dans lequel l'élément de mise en prise (132) comprend des premier et deuxième tampons (132) opposés configurés pour être déplacés l'un vers l'autre et en contact avec le fil-guide (105).

4. Dispositif de commande de fil-guide (100) selon la revendication 3, dans lequel les premier et deuxième dispositifs de préhension (134) opposés sont fixés aux premier et deuxième tampons (132) opposés respectivement, les premier et deuxième dispositifs de préhension (134) opposés étant configurés pour se déplacer entre la configuration de libération et une configuration de préhension, dans lequel lorsqu'ils sont dans la configuration de libération, les premier et deuxième dispositifs de préhension (134) opposés s'étendent radialement vers l'extérieur à partir d'une surface externe du boîtier (110), et lorsqu'ils sont dans la configuration de préhension, les premier et deuxième dispositifs de préhension (134) opposés sont disposés radialement vers l'intérieur vers le passage (114).

5. Dispositif de commande de fil-guide (100) selon la revendication 1, dans lequel l'interface (150) comprend une extrémité proximale (156) définissant une lumière (151) pour recevoir le fil-guide (105), l'extrémité proximale (156) ayant au moins une saillie (152) configurée pour tourner à l'intérieur d'au moins un canal (112) sur une surface interne du passage (114) du boîtier (110).

6. Dispositif de commande de fil-guide (100) selon la revendication 5, dans lequel l'interface (150) comprend une extrémité distale définissant une cavité (154) ayant un filetage interne (158) configuré pour recevoir une extrémité proximale extérieurement filetée (156) du raccord de cathéter (170).

7. Dispositif de commande de fil-guide (100) selon l'une quelconque des revendications 1 à 6, comprenant en outre un dispositif accessoire (200) comprenant un actionneur (210) ayant une lumière (214) configurée pour recevoir un cathéter (107), l'actionneur (210) ayant une extrémité distale définissant une pince pouvant être actionnée (211) configurée pour mettre en prise le cathéter (107), le dispositif accessoire (200) comprenant en outre un manchon (250) configuré pour actionner la pince pouvant être actionnée (211).

8. Dispositif de commande de fil-guide (100) selon la revendication 7, dans lequel la pince pouvant être actionnée (211) comprend une pluralité de bras pouvant être déviés (212) mobiles entre une configuration de libération, dans laquelle le cathéter (107) coulisse à travers la lumière (214), et une configuration de préhension, dans laquelle la pluralité de bras pouvant être déviés (212) mettent en prise le cathéter (107) et empêchent le cathéter (107) de se déplacer de manière axiale ou rotative.

9. Dispositif de commande de fil-guide (100) selon la revendication 8, dans lequel le manchon (250) définit une chambre proximale (252) configurée pour recevoir la pluralité de bras pouvant être déviés (212) dans la configuration de libération, une chambre centrale (254) configurée pour recevoir la pluralité de bras pouvant être déviés (212) dans la configuration de préhension, et une lumière (256) configurée pour recevoir le cathéter (107), dans lequel lorsque la pluralité de bras pouvant être déviés (212) de l'actionneur (210) est déplacée de la chambre proximale (252) dans la chambre centrale (254), la pluralité de bras pouvant être déviés (212) est comprimée radialement vers l'intérieur en mise en prise avec le cathéter (107).

10. Ensemble de commande de fil-guide (100, 200) comprenant :
le dispositif de commande de fil-guide (100) selon la revendication 1 ; et
un dispositif de serrage de cathéter (200) comprenant un premier élément (210) ayant une lumière (214) configurée pour recevoir un cathéter (107) et une extrémité distale définissant une pince pouvant être actionnée (211) configurée pour mettre en prise le cathéter (107), le dispositif de serrage de cathéter (200) comprenant en outre un deuxième élément (250) ayant une cavité (252, 254) configurée pour recevoir et actionner la pince pouvant être actionnée (211).

11. Ensemble de commande de fil-guide (100, 200) selon la revendication 10, dans lequel l'embrayage (130) comprend un élément de mise en prise (132) configuré pour saisir le fil-guide (105) et empêcher la rotation du fil-guide (105) alors que le boîtier distal (150) tourne, l'élément de mise en prise (132) comprenant des premier et deuxième tampons (132) opposés configurés pour être déplacés l'un vers l'autre et en contact avec le fil-guide (105).

12. Ensemble de commande de fil-guide (100, 200) selon la revendication 11, dans lequel l'embrayage (130) comprend en outre les premier et deuxième dispositifs de préhension (134) opposés fixés sur les premier et deuxième tampons (132) opposés, respectivement, les premier et deuxième dispositifs de préhension (134) opposés étant configurés pour se déplacer entre la configuration de libération et une configuration de préhension, dans lequel, lorsqu'ils sont dans la configuration de libération, les premier et deuxième dispositifs de préhension (134) opposés s'étendent radialement vers l'extérieur à partir d'une surface externe du boîtier proximal (110), et lorsqu'ils sont dans la configuration de préhension, les premier et deuxième dispositifs de préhension (134) opposés sont disposés radialement vers l'intérieur vers le passage (114).
